# EUROPÄISCHE PATENTSCHRIFT

(11) **EP 1 937 334 B1**
(45) Veröffentlichungstag und Bekanntmachung des Hinweises auf die Patenterteilung: **02.09.2009**
(21) Anmeldenummer: 06818578.4
(22) Anmeldetag: 16.11.2006
(51) Int. Cl.: A61M 5/30, A61M 5/31, A61M 5/24

(54) **INJEKTIONSVORRICHTUNG UND AMPULLENEINHEIT**
INJECTION DEVICE AND AMPOULE UNIT
DISPOSITIF D'INJECTION ET UNITE AMPOULE

(30) Priorität: 07.12.2005 DE 102005058655; 07.12.2005 DE 102005058656
(43) Veröffentlichungstag der Anmeldung: 02.07.2008
(60) Teilanmeldung: 09007392.5
(73) Patentinhaber: Painless Tech GmbH, 31134 Hildesheim (DE)
(72) Erfinder: LENZNER, Benedikt, 31134 Hildesheim (DE); THEUER, Stephan, 31319 Sehnde (DE); STÖRMER-TALLEUR, Bernd, 31249 Hohenhameln (DE); SCHULZ, Dirk, 68259 Mannheim (DE)
(74) Vertreter: Eisenführ, Speiser & Partner
(86) Internationale Anmeldenummer: PCT/EP2006/010990
(87) Internationale Veröffentlichungsnummer: WO 2007/065542

(56) Entgegenhaltungen:
- EP-A1- 0 367 677
- EP-A1- 0 595 508
- WO-A-96/15821
- WO-A-20/05021071
- CH-A5- 694 483
- DE-A1- 10 211 473
- GB-A- 2 404 865
- US-A- 4 124 024
- US-A- 5 137 516
- US-A- 5 891 086
- US-A1- 2003 050 596

## Beschreibung

Die Erfindung betrifft eine Injektionsvorrichtung zur nadelfreien Injektion, insbesondere subkutan, intrakutan, intramuskulär, intraartikulär, submucös, eines Mediums, mit einer Injektoreinrichtung und einer Ampulleneinheit.

In den letzten 10 bis 15 Jahren ist eine Vielzahl von Injektionsvorrichtungen zur nadelfreien Injektion eines Mediums entwickelt worden, wobei jedoch bis zum heutigen Zeitpunkt die Injektonsvorrichtungen die entsprechenden Ampulleneinheiten zerstören und unter Umständen beim Applizieren nicht verhindert werden kann, daß Materialpartikel unter hohem Druck mit dem zu applizierenden Medium auf oder unter die Haut gebracht wird, wobei durch die hohen Drücke und Geschwindigkeiten es häufig hierbei zu Verletzungen der Haut und des Gewebes kommen kann.

Beispielhaft sei hier auf die Vorrichtung verwiesen, die in DE 102 11 473 A1 offenbart ist, wobei es sich hierbei um eine Ampulle für ein Injektionsgerät zur nadellosen Injektion eines Mediums in ein menschliches oder tierisches Gewebe handelt, mit einem Ampullenkörper, mit einer innerhalb des Ampullenkörpers angeordneten Kammer zur Aufnahme des Mediums mit einer Düse zur Erzeugung eines Hochdruckstrahls des aus der Ampulle ausgestoßenen Mediums und einem längsbeweglichen Kolben und einem längsbeweglichen Stopfen zur Begrenzung der Kammer, wobei der Ampullenkörper einen von der Düse entfernten, aus einem mediumgerechten Material gefertigten Bereich und einem nahe der Düse angeordneten, durckstabilen Abschnitt aufweist, wobei ein Umströmbereich zur Umströmung des Stopfens durch das Medium auf dem der Kammer abgewandten Ende des druckstabilen Abschnittes angeordnet ist.

DE 695 08 104 T2 offenbart einen Glasbehälter für den Gebrauch als nadellose Injektionskapsel, welche einen hohlen Glaskörper aufweist, **dadurch gekennzeichnet, dass** Kompressionsmittel vorgesehen sind, um eine Kompressionskraft auf den hohlen Glaskörper auszuüben, um auf diese Weise den Behälter in die Lage zu versetzen, einen hohen Druck auszuhalten. Nachteilig an dieser Ausgestaltung ist jedoch die Tatsache, dass im vorderen konisch zuspitzend verlaufenden Bereich der Ampulle beim Applizieren sehr häufig Glassplitterbrüche auftreten, die bei der durchzuführenden Applikation dann zu kleinen Hautverletzungen führen.

Aus dem Stand der Technik ist das Dokument US 2003/0050596 A1 bekannt, eine nadellose Injektionseinrichtung mit einer vorgefüllten Ampulle.

Ferner wird als Stand der Technik auf das Dokument WO 96/15821 verwiesen, in welchem offenbart ist, dass ein Glascontainer, in welchem eine Flüssigkeit enthalten ist, außenseitig teilweise mit einer Kraft zusammengedrückt wird.

Das der Erfindung zugrundeliegende Problem besteht somit darin, eine entsprechende Injektionsvorrichtung sowie Ampulleneinheit bereitzustellen, die die oben erwähnten Nachteile nicht bzw. in stark abgeschwächter Form aufweisen, insbesondere darin, nahezu verletzungsfreie, nadelfreie Injektionen eines Mediums durch die Haut hindurch ausführbar zu gestalten.

Dieses Problem wird erfindungsgemäß durch eine Injektionsvorrichtung mit dem Merkmal nach Anspruch 1 gelöst. Vorteilhafte Weiterbildungen sind in den Unteransprüchen beschrieben.

Die erfindungsgemäße Injektionsvorrichtung zur nadelfreien Injektion eines Mediums weist eine Injektoreinrichtung und eine in Anspruch 1 nicht beanspruchte Ampulleneinheit auf. Die Ampulleneinheit wiederum weist einen das zu injizierende Medium aufbewahrenden Grundkörper sowie einen auf den Grundkörper Druckkräfte ausübenden Mantel auf, wobei der Mantel den Grundkörper in längsaxialer Richtung vollständig ummantelt. Erfindungswesentlich ist es unter anderem insbesondere, dass aufgrund der längsaxial vollständigen Ummantelung des Grundkörpers eine Glasbruch- und somit auch eine Verletzungsgefahr bei der Applizierung quasi ausgeschlossen ist.

Hierbei ist es vorteilhaft, dass die Druckkräfte des Mantels die bei der Herstellung des Grundkörpers entstandenen inneren Spannungen mindestens ausgleichen, da es sich bei den meisten Grundkörpern um solche handelt, die aus Glas hergestellt werden und bei der Glasherstellung eines ampullenförmigen Grundkörpers aufgrund der unterschiedlichen Abkühlung von außen nach innen entsprechend Spannungen aufgebaut werden, die abträglich sind. Beim Applizieren von Druckkräften auf den Grundkörper zum Austreiben des zu injizierenden Mediums wird somit die Wahrscheinlichkeit eines Bruches und dem Herauslösen von Grundkörperpartikeln vermindert, wobei es in diesem Zusammenhang besonders vorteilhaft ist, wenn der Mantel mindestens bis zu 100% der beim Injizieren auftretenden Druckkräften auf den Grundkörper ausübt, um somit eine vollständige Kompensierung und somit starke Herabsetzung einer Bruchgefahr zu bewerkstelligen.

Vorteilhafterweise wird/ist der Mantel auf den Grundkörper aufgeschrumpft, nach dem Castingverfahren, durch Polymerisation oder mittels Presspassung aufgebracht.

Das Schrumpfen erfolgt, indem der Mantel auf eine erhöhte Temperatur, beispielsweise auf +100°C, erwärmt wird, wobei sich dabei der Innendurchmesser ausdehnt und der Grundkörper eingeschoben werden kann. Beim Abkühlen, vorzugsweise auf Raumtemperatur, zieht sich der Mantel entsprechend wieder zurück und legt sich am Grundkörper an, wodurch der Mantel Druckkräfte auf den Grundkörper ausübt. Der gleiche Effekt wird erzielt, wenn anstatt der Erwärmung des Mantels der Grundkörper, beispielsweise in flüssigem Stickstoff, abgekühlt wird oder wenn gleichzeitig der Mantel erwärmt und der Grundkörper abgekühlt werden. Beim Castingverfahren wird insbesondere eine niedrig-schmelzende Metallegierung oder ein Kunststoff, beispielsweise Polyamid, mittels Sprühverfahren auf den Grundkörper aufgebracht. Hierbei kann beispielsweise und insbesondere der Grundkörper auf einem rotierenden Dorn angebracht sein, wobei gleichzeitig mittels einer Sprühvorrichtung die Legierung bzw. der Kunststoff aufgesprüht wird. Bei der Polymerisation wird ein Kunststoff auf den Grundkörper in einem Werkzeug aufgespritzt, wobei beispielsweise der Grundkörper sich in einer Spritzgußform befindet und in diese - also in den Hohlraum zwischen der Innenwandung des Spritzgußwerkzeugs und des Grundkörpers - entsprechend der jeweilig geeignete Kunststoff eingespritzt und somit auf den Grundkörper aufgespritzt wird. Bei der Presspassung wird der Grundkörper in einen Mantel, insbesondere aus Kunststoff oder einer Metallegierung oder einem Metall, mit Druck eingeschoben, wobei durch die etwas kleinere Innenpassung des Mantels gegenüber dem Außendurchmesser des Grundkörpers ein entsprechend großer Druck auf den Grundkörper aufgebaut wird.

Vorteilhafterweise, da in der Praxis bewährt, besteht der Mantel aus Kunststoff oder Metall, der Grundkörper aus Glas, insbesondere aus Borosilikatglas, da dieses eine ausgesprochen schwache Migrationsfähigkeit, einzelner Glasbestandteile also dem kontaminierendem Herauslösen von Bestandteilen, aufweist, und der Mantel insbesondere aus Polyamid, Faserverbundwerkstoff, insbesondere Kunststoffe mit Glasfasern und Kohlefasern, oder MCP-Legierung besteht. Bei den MCP-Legierungen handelt es sich um niedrig-schmelzende, metallische Legierungen, die insbesondere Bi, Sn und In enthalten, beispielsweise MCP 96- und MCP 137-Legierungen der Firma HEK GmbH, Kaninchenborn 24-28, D-23560 Lübeck.

Somit wird vorteilhafterweise ein aus Borosilikatglas bestehender Grundkörper mittels Aufschrumpfen bzw. mittels Castingverfahren durch Polymerisation oder Preßpassung von Polyamid, Faserverbundwerkstoff oder einer MCP-Legierung, welche ein starkes Schrumpfungsverhalten aufweisen, beim Abkühlen, vorteilhafterweise auf Raumtemperatur, und dem entstehenden Schrumpf und dem dadurch auftretenden Applizieren von hohen Druckkräften, der Grundkörper entsprechend stark beaufschlagt und somit beim Applizieren mittels hohen Drukkes zum Austreiben des zu applizierenden Mediums die Gefahr eines Materialbruches des Grundkörpers vermieden.

Mit erfindungswesentlich ist es unter anderem, daß durch das starke Schrumpfen bzw. das Castingverfahren bzw. Polymerisation bzw. Preßpassung die aufgebauten Druckkräfte sehr hoch sind und dadurch die Druckbeständigkeit beim Applizieren nicht mit herkömmlichem Kunststoff ummantelten Glasampullen zu vergleichen sind (so wie in DE 102 11 473 A1).

In diesem Zusammenhang ist es von Vorteil, wenn die Injektionsvorrichtung eine Injektorhülse zur nadelfreien Injektion eines Mediums aufweist, mit der Injektoreinrichtung, mit einem Federantriebselement zum Antreiben eines Treibelementes zum Austreiben des Mediums zum Injizieren desselben, wobei im Federspannungszustand die Injektoreinrichtung derart ausgebildet ist, daß hinsichtlich Federantriebselementspannung ausschließlich Federantriebselement, Treibelement, Sperrelement und Drucklagereinrichtung federspannungskraftbeaufschlagt sind, wobei durch die injektorhülsen-unabhängige Drucklagereinrichtung die Injektorhülse federspannungskräftefrei ist. Auf diese Weise ist es nicht notwendig, daß die Injektorhülse - wie sonst üblich - aus relativ kostspieligen Metallen bzw. faserverstärkten Kunststoffen ausgeführt werden muß, da erfindungsgemäß die Injektorhülse nunmehr keiner Federspannungskraftbeaufschlagung unterliegt.

Hierbei hat es sich in der Praxis als ausgesprochen vorteilhaft bewährt, daß über das Federantriebselement gespannte Treibelement über ein Sperrelement freigebbar arretiert/arretierbar ist und die durch das Federantriebselement erzeugten Kräfte durch eine Drucklagereinrichtung aufgenommen werden. Die Elemente Feder-antriebselement, Treibelement, Sperrelement sowie Drucklagereinrichtung bilden somit ein auf die Kräfte bezogenes vorgespanntes "geschlossenes System", unabhängig von den übrigen Teilen der Vorrichtung.

Aufgrund eines geringen Platzbedarfes und einer in längsaxialer Richtung gesehenen schlanken Ausführbarkeit ist es von Vorteil, wenn das Sperrelement ein stiftartiges Element ist, das am Treibelement dieses arretierend angreift. In diesem Zusammenhang ist es von Vorteil, wenn das Sperrelement als Hebel ausgestaltet ist und somit über eine entsprechend gewünschte leichte Gängigbarkeit über die entsprechenden Hebelverhältnisse die Auslösekräfte dann entsprechend einstellbar sind und/oder das Treibelement an einer Hinterschneidung arretiert.

In diesem Kontext ist es weiterhin von Vorteil, wenn ein Auslöseelement zum Freigeben des über das Federantriebselement gespannten Treibelementes im wesentlichen in Injektionsrichtung des Mediums betätigbar ist, um beim Injizieren durch die Haut einen von der medizinischen Indikation abhängigen günstigen Applikationswinkel zur Hautoberfläche einzustellen und eine entsprechend leichte Betätigbarkeit aufzuweisen. Vorteilhaft ist in diesem Zusammenhang auch, daß die Freigabe des Treibelementes durch Verschwenken oder Verdrehen des Sperrelementes bewerkstelligbar ist, wobei in diesem Zusammenhang es darüber hinaus vorteilhaft ist, wenn das Auslöseelement keilartig ausgestaltet ist, das beim Betätigen des Auslöseelementes im wesentlichen in Injektionsrichtung auf das Sperrelement aufläuft.

Weiterhin ist es optional vorteilhaft, wenn die Ampulleneinheit an der Injektoreinrichtung auf- und/oder abmontierbar ist, insbesondere die Ampulleneinheit kraftschlüssig an der Injektoreinrichtung die Injektionsvorrichtung in einen Präinjektionszustand bringend aufmontierbar ist, um somit erst vor der Applikation teilweise empfindliche Medien an die Injektoreinrichtung zu bringen, um anschließend ohne vorher eventuell auftretende Zersetzungsprozesse zu applizieren. Im wesentlichen ist jedoch somit mittels der Injektoreinrichtung und entsprechenden Ampulleneinheiten durch Zusammenbau zur erfindungsgemäßen Injektionsvorrichtung es möglich, unterschiedliche Medien mit einer Injektoreinrichtung zu applizieren.

In diesem Zusammenhang ist es vorteilhaft, wenn die Auf- und Abmontierbarkeit mittels eines Gewindes oder einer ringförmigen Rastverbindung realisiert ist, welche Ausführungsformen sind, die sich in der Praxis bewährt haben.

Darüber hinaus ist von Vorteil, wenn die Ampulleneinheit mit dem zu injizierenden Medium vorbefüllt, insbesondere nahezu gasfrei oder gasfrei, ist, so daß beim Injizieren durch Blasenbildung keine Druckverluste entstehen bzw. eventuell Luft oder andere Gase beispielsweise durch Stickstoff mit appliziert werden, was unter Umständen zu gefährlichen Embolien im Körper führen könnte.

Um ein unbeabsichtigtes Auslösen der Injektionsvorrichtung zu verhindern, weist dieses vorteilhafterweise ein Sicherungselement zum Sichern des Auslöseelementes auf, wobei dieses insbesondere ringartig ausgestaltet ist und weiterhin insbesondere die Injektoreinrichtung und/oder Auslöseelement eine Nut zur Aufnahme des Sicherungselementes aufweist, so daß durch einfaches An- bzw. Abclipsen, also Abziehen bzw. Aufstecken das Auslöseelement beim Transport und/oder gegen unbeabsichtigtes Auslösen gesichert ist.

Vorteilhafterweise weist die Ampulleneinheit ein eine längsaxiale Seite abschließendes, längsbewegliches Stopfenelement auf, welches vorteilhafterweise beim Injizieren das Medium aus der Ampulleneinheit austreibt.

Vorteilhafterweise ist die Ampulleneinheit kraftschlüssig an einer Injektoreinrichtung, insbesondere der erfindungsgemäßen Injektoreinrichtung, in einem Präinjektionszustand bringend, aufmontierbar, wobei vorteilhafterweise die Auf- und Abmontierbarkeit mittels eines Gewindes oder einer ringförmigen Rastverbindung realisiert ist.

Weiterhin ist es von Vorteil, wenn am längsaxialen Ende der Ampulleneinheit als Austritt des zu injizierenden Mediums eine Düse, insbesondere aus Stahl insbesondere bestehend aus der Legierung 1.4301 oder 1.4435, wobei es sich bei der einen um einen austenitischen, nicht rostenden Stahl, im Kurznamen X5CrNi18 10 (AISI 304), sonstige gebräuchliche geschützte Bezeichnungen V2A, mit der chemischen Zusammensetzung C≤0,07%, Si≤1,0%, Mn≤2,0%, Cr=17 bis 20%, Ni=8,5 bis 10%, bzw. es sich um einen austenitischen, nichtrostenden Stahl, mit dem Kurznamen X2CrNiMo 18 14 3 (316 L), mit der geschützten Bezeichnung SUPRA und der chemischen Zusammensetzung von C≤0,03%, Si≤1,0%, Mn≤2,0%, Cr=17,0 bis 18,5%, Mo=2,5 bis 3,0%, Ni=12,5 bis 15,0%, handelt, mit einer oder mindestens zwei Austrittsöffnungen, angeordnet ist, um beim Applizieren einen entsprechenden Medium-Hochdruckstrahl zu erhalten. Bei mehreren Austrittsdüsen ist aufgrund der größeren Applikationsfläche eine indikationsabhängige Kinetik einstellbar.

Weiterhin ist es vorteilhaft, wenn das längsaxiale Ende der Ampulleneinheit als Austritt des zu injizierenden Mediums von einer Kappe abgedeckt ist, um sterile Bedingungen bis kurz vor der Applikation bereitzustellen.

Schließlich ist es vorteilhaft, wenn zwischen dem Grundkörper und der Düse ein elastisches Dichtglied, insbesondere aus Silikon bestehend, angeordnet ist, um beim Applizieren zu verhindern, daß das Medium teilweise sich zwischen Grundkörper und Stahldüse schiebt und aufgrund eines Druckabfalles und/oder einer Leckage eine wesentlich schlechtere oder keine Applizierbarkeit gegeben wäre.

Schließlich ist es vorteilhaft, wenn sowohl bei der erfindungsgemäßen Injektionsvorrichtung als auch bei der Ampulleneinheit die Düse mit der Düsenverschraubung einen dichtenden Paßsitz bildet, insbesondere, wenn bei im Längsschnitt gesehen konisch zuspitzend verlaufender innerer Ausgestaltung der Düsenverschraubung im zusammengebauten Zustand diese sich an die äußere Oberfläche der auch im Längsschnitt gesehenen konisch zulaufenden Düse anschmiegt, da auf diese relativ einfache Art und Weise und doch sehr effizient eine hohe Dichtigkeit erreicht wird und somit die Düse quasi in der Düsenverschraubung dichtend und passend sitzt. Darüber hinaus wird durch den gebildeten Paßsitz die Düse entsprechend wunschgemäß ausgerichtet und liegt entsprechend wunschgemäß am Dichtelement dichtend an. Auf diese Weise ist sowohl eine Ausrichtung als auch Dichtung bewerkstelligt.

Darüber hinaus ist es vorteilhaft, wenn eine das Stopfenelement antreibende Kolbenstange hohl ist und insbesondere darüber hinaus das Stopfenelement eine zur Hohlheit der Kolbenstange korrespondierende Materialausnehmung aufweist, da auf diese Weise eine bequeme Befüllung des Grundkörpers mit dem später zu applizierenden Medikament bewerkstelligt werden kann durch Einführen eines spritzenartigen Applikators einer üblichen Befüllungsanlage durch den hohlen Kanal der Kolbenstange und die Materialausnehmung des Stopfenelementes hindurch, um anschließend eine bestimmte Menge eines entsprechenden Medikamentes in den Grundkörper zu überführen, um anschließend beispielsweise und insbesondere nach Herausfahren des nadelförmigen Applikators die hohle Kolbenstangen ein oder mehrere Male zu quetschen, um somit die dann gebildete Ampulle nahezu gasfrei bzw. gasfrei befüllt zu haben.

Weiterhin ist es von Vorteil, wenn das Stopfenelement nach Auslösen, also insbesondere beim Implizieren eines bestimmten Medikaments durch die Haut, bei Zurückziehen der Kolbenstange sich von der Kolbenstange löst, um auf diese Weise eine unerwünschte Mehrfachverwendung zu vermeiden. Beispielsweise und insbesondere kann eine solche Ausgestaltung dadurch verwirklicht sein, daß der vordere Teil der Kolbenstange gegen den hinteren Teil des Stopfenelementes anschlägt, jedoch nicht durch Hintergreifen der Elemente beim Rückziehen gesichert ist, so daß die Kolbenstange sich frei nach hinten bewegen kann ohne das Stopfenelement mitzunehmen.

Schließlich ist es von Vorteil, wenn nach Betätigen des Auslöseelementes dieses in der Injektorhülse verbleibt, um auf diese Weise eine unerwünschte Mehrfachbetätigung zu vermeiden. Dies kann beispielsweise und insbesondere dadurch verwirklicht werden, daß sich das Sperrelement beim Auslösen von einer Hinterschneidung des Treibelementes löst, quasi "abfällt", und lose im Inneren des Injektors liegt.

Schließlich ist es weiterhin vorteilhaft, wenn die Kolbenstange mindestens einmal, vorteilhafterweise zweimal, dichtend abgequetscht ist.

Die Injektionsvorrichtung und die Ampulleneinheit stellen keinen Sondermüll dar, im Gegensatz zu mit Gewebe, Blut, Blutbestandteilen oder einem anderen infektiösen Material in Kontakt gekommenen Kanülen von herkömmlichen Spritzen.

Die im Zusammenhang mit der erfindungsgemäßen Injektionsvorrichtung obig dargestellten Merkmale hinsichtlich der Ampulleneinheit und deren Vorteile gelten selbstverständlich auch für die Ampulleneinheit selbst.

Die Erfindung wird im nachfolgenden beispielhaft dargestellt und näher erläutert, wobei die nachfolgenden Figuren folgendes darstellen:
- Fig. 1: ist eine skizzenhafte Querschnittsdarstellung einer Ampulleneinheit gemäß einer ersten Ausführungsform;
- Fig. 2: ist eine skizzenhafte Querschnittsansicht einer zweiten Ausführungsform der Ampulleneinheit;
- Fig. 3: ist eine explosionsartige Querschnittsdarstellung der in Fig. 1 dargestellten Ampulleneinheit;
- Fig. 4: ist eine skizzenhafte Querschnittsansicht des vorgespannten "geschlossenen Systems" in einer Injektoreinrichtung der erfindungsgemäßen Injektionsvorrichtung;
- Fig. 5: ist eine explosionsartige skizzenhafte Querschnittsdarstellung einer Injektoreinrichtung der erfindungsgemäßen Vorrichtung mit gespanntem Injektor;
- Fig. 6: ist eine explosionsartige skizzenhafte Querschnittsdarstellung der in Fig. 5 gezeigten Injektoreinrichtung im ungespannten Zustand;
- Fig. 7 a) und b): zeigen das grundsätzliche Auslöseprinzip zum Injizieren eines Mediums bei der erfindungsgemäßen Injektionsvorrichtung.
- Fig. 8: ist eine skizzenhafte Querschnittsdar stellung einer Ampulleneinheit gemäß einer weiteren Ausführungsform;
- Fig. 9: ist eine explosionsartige Querschnittsdarstellung der in Fig. 8 dargestellten Ampulleneinheit;
- Fig. 10a bis c: zeigen skizzenhaft das Funktionsprinzip des selbstabwerfenden Stopfenelementes sowie der Abdichtung der Kolbenstange.

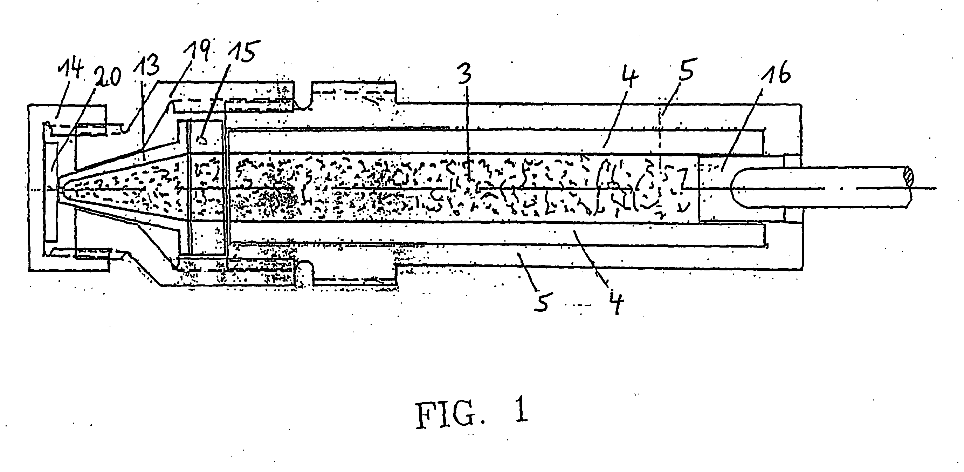

In Fig. 1 ist skizzenhaft im Querschnitt eine Ampulleneinheit dargestellt, die an einem ihrer längsaxialen Enden ein Stopfenelement 16 (mit Kolbenstange, wie angedeutet) aufweist. Am anderen längsaxialen Ende des mit einem Mantel 5 umgebenen, mit einem Medium 3 gefüllten Grundkörpers 4, der vom Mantel 5 in längsaxialer Richtung vollständig ummantelt ist, befindet sich zwischen einer Stahldüse 13 und dem Grundkörper 4 bzw. Mantel 5 ein Dichtelement 15 aus Silikon, wobei die Stahldüse 13 kraft- und formschlüssig mittels einer Düsenverschraubung 19 mechanisch und mediumabdichtend fixiert ist. Am Austritt der Stahldüse 13 befindet sich aufgeschraubt eine Kappe 14, wobei zwischen Innenwand der Kappe 14 und Austritt der Stahldüse 13 sich wiederum eine Kappendichtung 20 (aus sterilen Gründen) befindet. Vor dem Injizieren wird eine solche Ampulleneinheit an eine Injektoreineinrichtung angebracht, insbesondere aufgeschraubt, um nach Entfernen der Kappe 14 und der Kappendichtung 20 nach Aufsetzen auf ein bestimmtes Hautareal das Medium 3 zu applizieren durch zumindest teilweises längsaxiales Verschieben des Stopfenelementes über die angetriebene Kolbenstange von rechts nach links.

Die Düse 13 wird über den mit der Innenwandung der Düsenverschraubung 19 gebildeten Paßsitz in Längsrichtung zentriert ausgerichtet und liegt dann folglich plan am Dichtelement 15 dichtend an, so daß bei Applikation das Medium 3 verlustfrei und zielgerichtet appliziert werden kann.

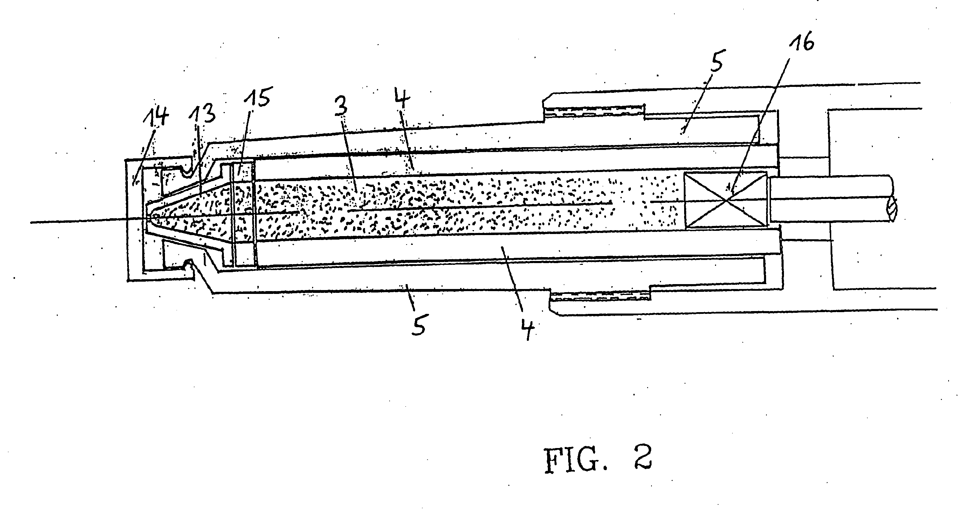

In Fig. 2 ist eine Variante zu der in Fig. 1 gezeigten Ausführungsform der Ampulleneinheit gezeigt, wobei diese sich im wesentlichen darin unterscheidet, daß statt einer Düsenverschraubung 19 der Mantel derart geformt ist, daß die Stahldüse 13 form- und kraftschlüssig an den Innenwandungen des Mantels 5 anliegt und einen Paßsitz bildet. Ein weiterer Unterschied besteht darin, daß die Kappe 14 keine zusätzliche Kappendichtung aufweist und diese nicht abschraubbar sondern absteckbar ist.

In den Fig. 1 und 2 ist für den Fachmann eindeutig zu erkennen, daß in längsaxialer Richtung der Grundkörper 4 vollständig vom Mantel 5 ummantelt ist.

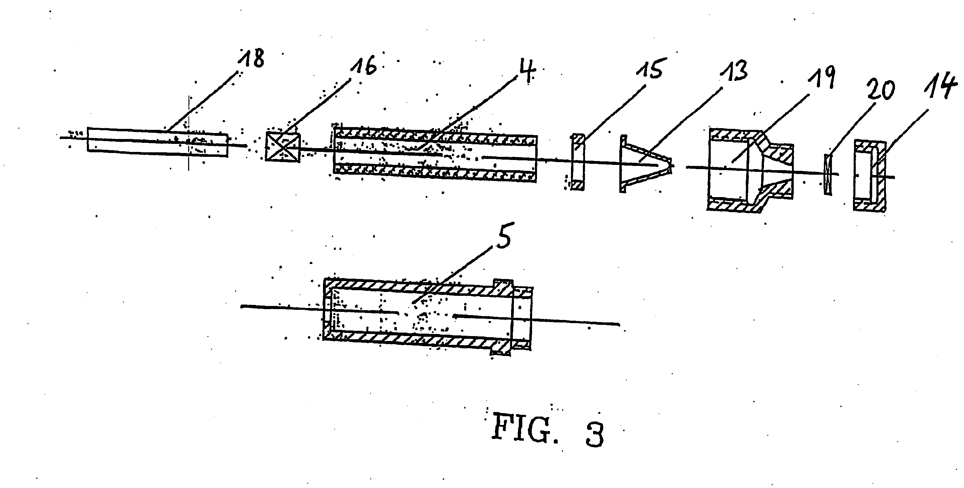

In Fig. 3 ist in einer explosionsartigen Darstellung im Querschnitt die in Fig. 1 gezeigte Ampulleneinheit dargestellt, so daß die einzelnen Bestandteile nochmals klar zu erkennen sind, nämlich die das Stopfenelement 16 antreibende Kolbenstange 18, das in längsaxiale Richtung im Grundkörper 4 verschiebliche Stopfenelement 16, den das Medium 3 aufnehmenden Grundkörper 4, den den Grundkörper 4 ummantelnden Mantel 5, die die Dichtigkeit zwischen Stahldüse 13 und Grundkörper 4 bzw. Mantel 5 herstellende Dichtung 15, vorzugsweise aus Silikon, die eine winzige Austrittsöffnung aufweisende und damit quasi beim Verfahren des Stopfenelementes 16 Medium-Hochdruckstahl erzeugende Stahldüse 13, die Stahldüse 13 und das Dichtelement 15 am Grundkörper 4 bzw. am Mantel 5 form- und kraftschlüssige bereitstellende Fixierung über die Düsenverschraubung 19 sowie die weiter abdichtende Kappendichtung 20 und schließlich die entsprechende Kappe 14, die beide aus sterilen Gründen erst kurz vor der Applizierung abgenommen werden.

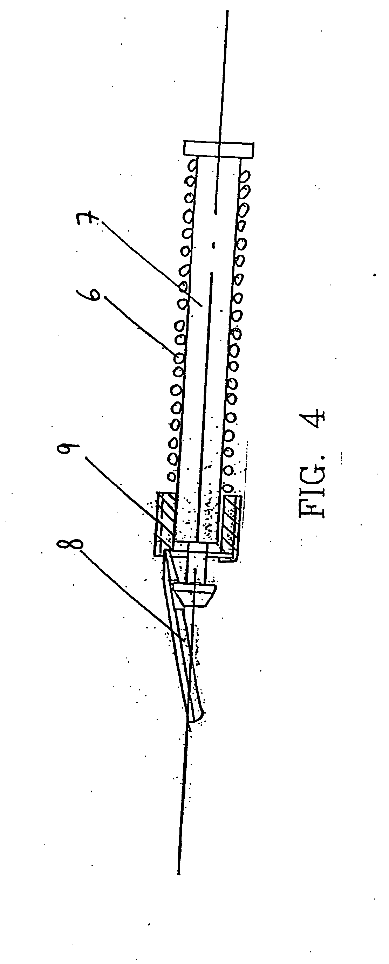

In Fig. 4 sind skizzenhaft im Querschnitt die das vorgespannte geschlossene System in der Injektoreinrichtung der erfindungsgemäßen Injektionsvorrichtung bildenden Elemente dargestellt, wobei das über das Federantriebselement 6 gespannte Treibelement 7 über ein Sperrelement 8 freigebbar arretiert ist mittels einer Materialausnehmung, wobei die durch das Federantriebselement 6 erzeugten Kräfte durch eine Drucklagereinrichtung 9 aufgenommen werden. Diese vier Elemente stellen das vorgespannte "geschlossene System" dar.

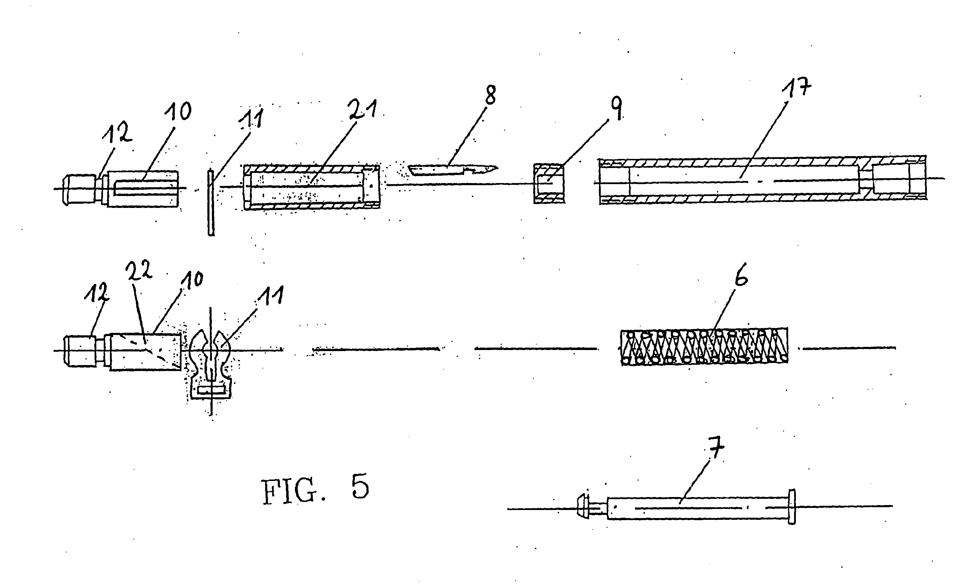

In Fig. 5 ist in explosionsartiger Querschnittsdarstellung der strukturelle Aufbau der Injektoreinrichtung der erfindungsgemäßen Injektionsvorrichtung dargestellt. Ein stielartiges Auslöseelement 10 weist eine Nut 12 zur Aufnahme eines halbringförmigen Sicherungselementes 11 zum Sperren und Verhindern eines unbeabsichtigten Auslösens, auch beim Transport, auf, wobei sich in einer Abdeckkappe 21 der Injektoreinrichtung ein stiftförmiges Sperrelement 8 mit einer Materialausnehmung befindet, welches auf dem Drucklager arretiert ist, so daß durch Längsverschieben des Auslöseelementes 10 und damit des Innenkeils 22 dieser auf das Sperrelement 8 aufläuft und über die Hebelverhältnisse und Verschwenkbarkeit bzw. Verdrehbarkeit das rechte und der Drucklagereinrichtung 9 zugewandte Ende des Sperrelementes 8 das linke Ende des Treibelementes 7 entarretierend freigibt, so daß dieses aufgrund des vorgespannten Federantriebselementes 6 längsaxial nach rechts und somit eine Kolbenstange einer anmontierten Ampulleneinheit auch nach rechts und somit das Medium aus dem Grundkörper 4 der Ampulleneinheit austreibend verschoben wird.

Das Verschieben des Treibelementes 7 findet in einer Injektorhülse 17 der Injektoreinrichtung statt.

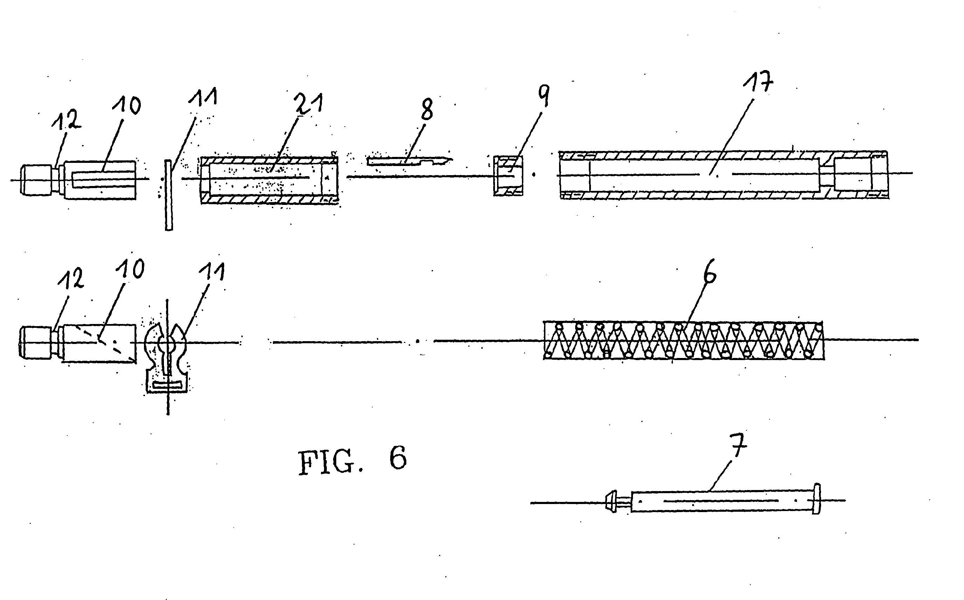

Fig. 6 zeigt die in Fig. 5 dargestellte Ausführungsform einer Injektoreinrichtung der erfindungsgemäßen Injektionsvorrichtung, lediglich jedoch in einem ungespannten Zustand.

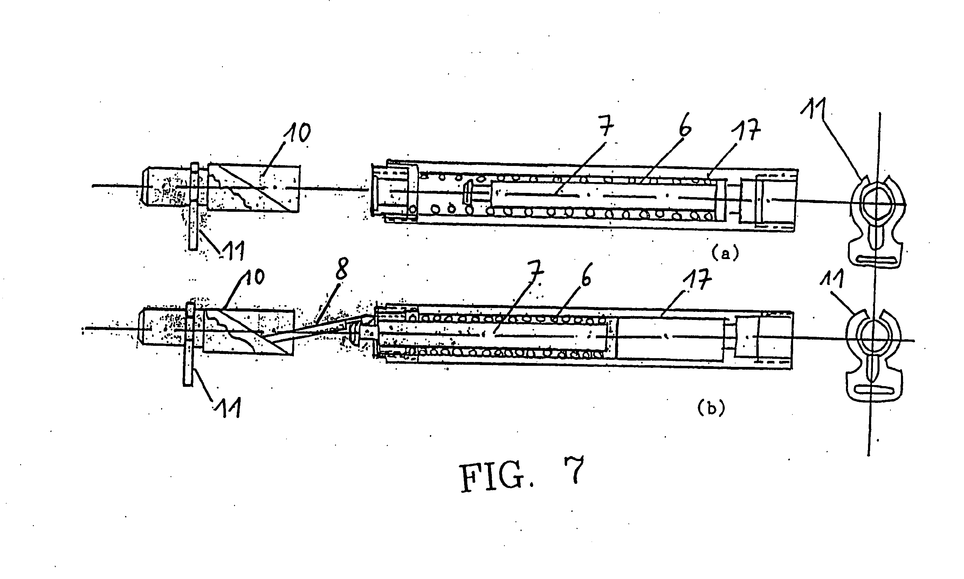

In den Fig. 7 a) und 7 b) sind die funktionellen Zusammenhänge, die wie zu den Figuren 5 und 6 schon obig erläutert, nochmals zur weiteren Klarstellung dargestellt, wobei es sich bei der in Fig. 7 a) gezeigten um eine entspannte und bei Fig. 7 b) gezeigten um eine gespannte Injektionsvorrichung handelt.

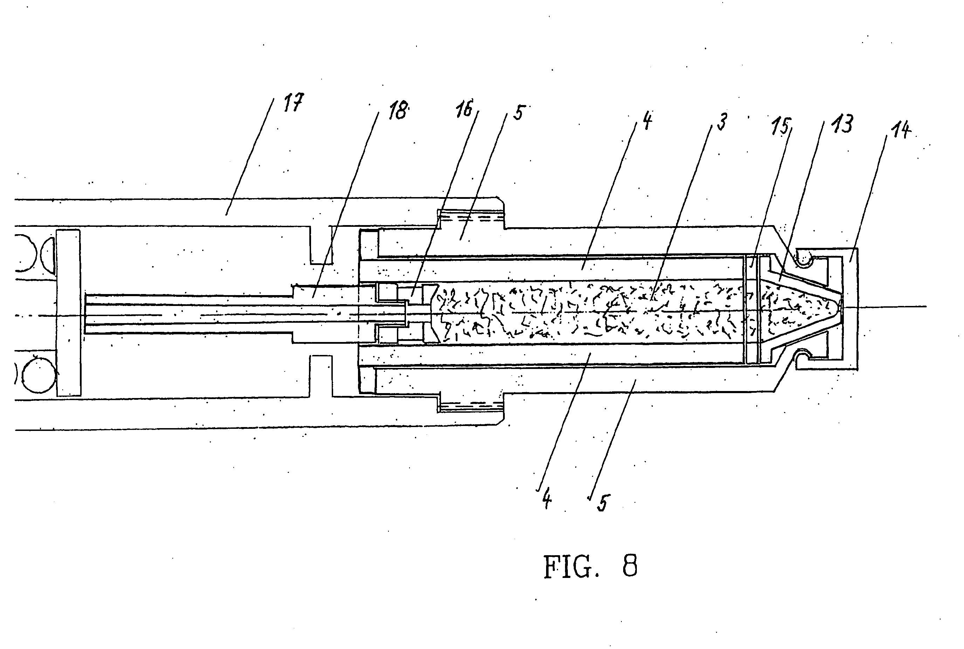

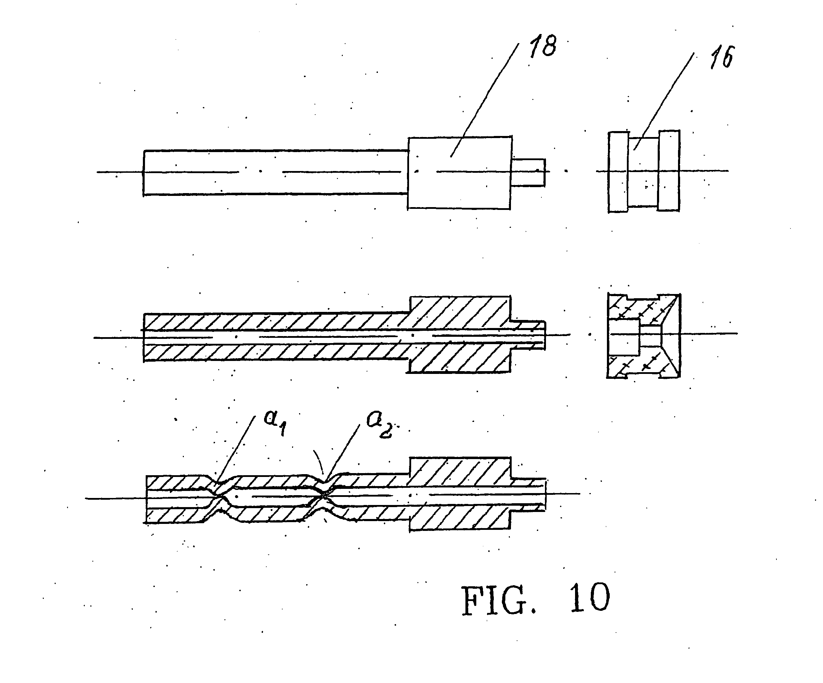

In Fig. 8 ist im Querschnitt skizzenhaft eine weitere Ausführungsform der Ampulleneinheit zu erkennen, bei der ein in längsaxialer Richtung durchgängiger Mantel 5 vollständig einen aus Borosilikatglas bestehenden Grundkörper 4 in längsaxialer Richtung ummantelt, wobei das vordere Ende des Grundkörpers 4 an einem scheibenförmigen Dichtelement 15 abdichtend anliegt, das wiederum dichtend an einer Düse 13 anstößt. Die Düse 13 selbst läuft auf den sich konisch verjüngenden Teil der Innenwandung der Düse dichtend auf, dadurch daß über das durch den entstandenen Paßsitz gegebene Widerlager ein Auslaufen des sich im Grundkörper 4 befindenen Mediums 3 der zu injizierenden Lösung über die abdichtende Wirkung des Dichtelementes 15 verhindert wird. Die aus Metall bestehende Düse 13 wird über eine an dem vorderen Bereich des Mantels angeordneten Hintergreifungen eingreifbare/einklippbare Kappe 14 abnehmbar verschlossen. Am hinteren längsaxialen Endes des Grundkörpers 4 ist ein längsaxial bewegliches Stopfenelement 16 angeordnet, das aus Silikon besteht, so daß auch in dieser Richtung beim längsaxialen Verfahren zum Entleeren des Grundkörpers 4 sich eine Dichtung an den inneren Glaswandungen des Grundkörpers 4 ausbildet aufgrund der reversiblen Verformbarkeit des Silikonmaterials. Das Stopfenelement 16 wird durch eine anschlagende hohle Kolbenstange 18 angetrieben, wobei die Hohlheit der Kolbenstange 18 korrespondiert mit einer Materialausnehmung des Stopfenelementes 16 (s. auch Fig. 10), so daß eine über einen automatischen nadelförmigen Applikator durch Einführen durch die hohle Kolbenstange und das Stopfenelement hindurch eine Befüllung mit dem Medium 3 ermöglicht wird. Nach entsprechender Entleerung und Zurückziehen der Kolbenstange 18 löst sich das Stopfenelement 16 von der Kolbenstange 18, da diese lediglich in Entleerungsrichtung gegen das Stopfenelment 16 auflief und dann nunmehr freigegeben wird.

Die Injektorhülse 17 umgreift zumindest teilweise den Mantel 5.

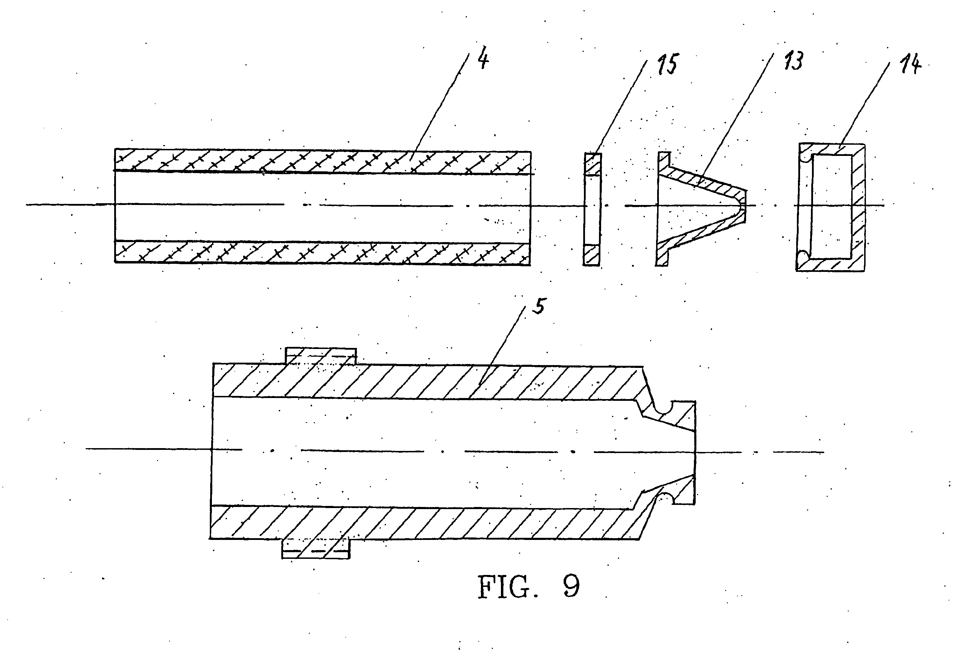

In Fig. 9 ist skizzenhaft das Funktionsprinzip der Abdichtung durch Zusammenwirken des Grundkörpers 4, des Dichtelementes 15, der Düse 13 sowie der Kappe 14 in Verbindung mit dem Mantel 5 dargestellt. Die Abdichtung erfolgt durch das klemmende Auflaufen und dadurch einen dichtenden Paßsitz bildend der Düse 13 auf den inneren konisch zulaufenden Teil des Mantels 5 und weiterer Wechselwirkung der Düse 13 mit dem Dichtelement 15 und dem Grundkörper 4.

In Fig. 10a und b ist das Wechselwirkungsprinzip zwischen Kolbenstange 18 und Stopfenelement 16 aufgezeigt, wobei klar zu erkennen ist, daß ein vorderer Teil der Kolbenstange 18 beim Injizieren gegen einen hinteren Teil des Stopfenelementes 16 drückt und in umgekehrter Richtung freigebbar ist.

In Fig. 10c ist eine zweifach abgequetschte hohle Kolbenstange 18 zu erkennen, die nach einer entsprechenden Befüllung mit einem diesbezüglichen Mediums entsprechend zusammenquetschend abgedichtet worden ist, so daß das abgefüllte Medium steril aufbewahrt werden kann.

Allgemein läßt sich noch folgendes feststellen: Die Düse 13, das elastische Dichtelement 15 sowie der Grundkörper 4 werden mit dem Mantel 5 zusammengebaut, um anschließend die Kappe 14 auf dem Mantel 5 aufzuschrauben. Das Stopfenelement 16 wird mit der Kolbenstange 18 zusammengesteckt und danach in den Grundkörper 4 der Ampulleneinheit eingeschoben. Alle Komponenten können aufgrund der besonderen Konstruktion des Stopfenelementes 16 und der Kolbenstange 18 unter normalen Bedingungen zusammengebaut werden; d. h. daß kein Reinraum erforderlich ist. Anschließend werden die kompletten Ampulleneinheiten in sogenannte Trays zu 20*20 Stück gestellt, wobei jedes Tray in einzelne Polyethylenbeutel verpackt wird. Eine Vielzahl an Trays wird auf eine Palette gegeben und nochmals verpackt. Die gesamte Palette wird anschließend mit Gammastrahlen sterilisiert. Anschließend werden die Trays im Sterilraum aus dem Polyethylenbeutel entnommen und einer Abfüllanlage zugeführt, wobei die Abfüllanlage die Ampulleneinheiten durch die hohle Kolbenstange und dem Stopfenelement mit entsprechender Materialausnehmung, insbesondere und beispielsweise Bohrungen, befüllt. Danach wird die Kolbenstange zuerst an einer vom eigentlichen Grundkörper 4 entfernteren Stelle A1 und anschließend an einer diesbezüglich näheren Stelle A2 abgequetscht. Durch dieses Verfahren ist der Inhalt der Ampulleneinheit nahezu gasfrei bzw. gasfrei.

Das Besondere an dieser Erfindung ist die Tatsache, daß Medikamente nunmehr für eine lange Zeit (> 30 Tage) für ein nadelloses Spritzen-Applikationssystem vorgehalten werden können.

## Patentansprüche

1. Injektionsvorrichtung zur nadelfreien Injektion eines Mediums, mit einer Injektoreinrichtung und einer Ampulleneinheit, wobei die Ampulleneinheit (2) einen das zu injizierende Medium (3) aufbewahrenden Grundkörper (4) aufweist, der einen auf den Grundkörper (4) Druckkräfte ausübenden Mantel (5) aufweist,
wobei am längsaxialen Ende der Ampulleneinheit als Austritt des zu injizierenden Mediums (3) eine Düse (13) angeordnet ist, welche in Verlängerung des Grundkörpers liegt,
**dadurch gekennzeichnet, dass** der Mantel (5) den Grundkörper (4) in Längsaxialrichtung vollständig und die Düse (13) teilweise umschließt, so dass die Düse (13) form- und kraftschlüssig an den Innenwandungen des Mantels (5) anliegt und einen Passsitz bildet, dass die Druckkräfte des Mantels (5) die bei der Herstellung des Grundkörpers entstehenden inneren Spannungen mindestens ausgleichen, der Grundkörper aus Glas, vorzugsweise Borosilikatglas, besteht, dass die Kolbenstange (18) hohl ist und nach dem Befüllen der Ampulle mit dem Medium die Kolbenstange mindestens an einer Stelle abgequetscht ist.

2. Injektionsvorrichtung nach Anspruch 1,
**dadurch gekennzeichnet, dass** der Mantel (5) mindestens bis zu 100 % der beim Injizieren auftretenden Druckkräfte auf den Grundkörper ausübt.

3. Injektionsvorrichtung nach einem der Ansprüche 1 bis 2,
**dadurch gekennzeichnet, dass** der Mantel (5) auf den Grundkörper (4) aufgeschrumpft oder nach dem Castingverfahren, durch Polymerisation oder mittels einer Presspassung aufgebracht wird/ist.

4. Injektionsvorrichtung nach einem der Ansprüche 1 bis 3,
**dadurch gekennzeichnet, dass** der Mantel (5) aus Kunststoff oder Metall besteht, der Grundkörper (4) bevorzugt aus Glas besteht und der Mantel (5) bevorzugt aus Polyamid, Faserverbundwerkstoff oder MCP-Legierung besteht.

5. Injektionsvorrichtung nach einem der Ansprüche 1 bis 4,
**gekennzeichnet durch** eine Injektorhülle (17) zur nadelfreien Injektion eines Mediums (3), mit der Injektoreinrichtung mit einem Federantriebselement (6) zum Antreiben eines Treibelements (7) zum Austreiben des Mediums (3) zum Injizieren desselben, wobei im Federspannungszustand die Injektoreinrichtung derart ausgebildet ist, dass hinsichtlich Federantriebselementspannung ausschließlich Federantriebselement (6), Treibelement (7), Sperrelement (8) und Drucklagereinrichtung (9) federspannungskraftbeaufschlagt sind, wobei **durch** die injektorhülsen-unabhängige Drucklagereinrichtung (9) die Injektorhülse (17) federspannungskräftefrei ist, das vorzugsweise über das Federantriebselement (6) gespannte Treibelement (7) über ein Sperrelement (8) freigebbar arretiert/arretierbar ist und die **durch** das Federantriebselement (6) erzeugten Kräfte durch eine Drucklagereinrichtung (9) aufgenommen werden und weiter vorzugsweise das Sperrelement (8) ein stiftartiges Element ist, das am Treibelement (7) dieses arretierend angreift und vorzugsweise das Sperrelement (8) als Hebel ausgestaltet ist und/oder das Treibelement (7) an einer Hinterschneidung arretiert und bevorzugt ein Auslöseelement (10) zur Freigeben des über das Federantriebselement (6) gespannten Treibelementes (7) in Injektionsrichtung des Mediums (3) betätigbar ist, dass vorzugsweise die Freigabe des Treibelements (7) **durch** Verschwenken oder Verdrehen des Sperrelementes (8) bewerkstelligbar ist, dass vorzugsweise das Auslöseelement (10) keilartig ausgestaltet ist, dass beim Betätigen des Auslöseelements (10) in Injektionsrichtung dieses auf das Sperrelement (8) aufläuft.

6. Injektionsvorrichtung nach einem der Ansprüche 1 bis 5,
**dadurch gekennzeichnet, dass** die Ampulleneinheit an der Injektoreinrichtung auf- und/oder abmontierbar ist, dass vorzugsweise die Ampulleneinheit kraftschlüssig an der Injektoreinrichtung die Injektionsvorrichtung in einen Präinjektionszustand bringend aufmontierbar ist, dass vorzugsweise die Auf- und Abmontierbarkeit mittels eines Gewindes oder einer ringförmigen Rastverbindung realisiert ist.

7. Injektionsvorrichtung nach einem der Ansprüche 1 bis 6,
**dadurch gekennzeichnet, dass** die Ampulleneinheit mit dem zu injizierenden Medium vorbefüllt ist, dass vorzugsweise die Ampulleneinheit nahezu gasfrei oder gasfrei vorbefüllt ist.

8. Injektionsvorrichtung nach einem der Ansprüche 1 bis 7,
**dadurch gekennzeichnet, dass** die Injektoreinrichtung ein Sicherungselement (11) zum Sichern des Auslöseelementes (10) beim Transport und/oder gegen unbeabsichtigtes Auslösen aufweist, dass vorzugsweise das Sicherungselement (11) ringartig ausgestaltet ist, dass vorzugsweise die Injektoreinrichtung und/oder das Auslöseelement (10) eine Nut (12) zur Aufnahme des Sicherungselementes (11) aufweist, dass vorzugsweise die Injektionsvorrichtung ein eine längsaxiale Seite abschließendes, längsbewegliches Stopfenelement (16) aufweist, dass vorzugsweise das Stopfenelement (16) beim Injizieren das Medium aus der Ampulleneinheit austreibt, dass vorzugsweise die Auf- und Abmontierbarkeit mittels eines Gewindes oder einer ringförmigen Rastverbindung realisiert ist, dass vorzugsweise am längsaxialen Ende der Ampulleneinheit als Austritt des zu injizierenden Mediums (3) eine Düse (13) angeordnet ist, dass vorzugsweise das längsaxiale Ende der Ampulleneinheit als Austritt des zu injizierenden Mediums (3) von einer Kappe (14) abgedeckt ist, dass vorzugsweise zwischen Grundkörper (14) und Düse (13) ein elastisches Dichtglied (15) angeordnet ist, dass vorzugsweise das elastische Dichtglied (15) aus Silikon besteht, dass vorzugsweise die Düse (13) aus Stahl besteht, dass vorzugsweise die Düse (13) eine oder mindestens zwei Austrittsöffnungen aufweist, dass vorzugsweise die Düse (13) mit der Düsenverschraubung (19) einen dichtenden Passsitz bildet, dass vorzugsweise eine das Stopfenelement (16) antreibende Kolbenstange (18) hohl ist, dass vorzugsweise das Stopfenelement (16) eine zur Hohlheit der Kolbenstange (18) korrespondierende Materialausnehmung aufweist, dass vorzugsweise das Stopfenelement (16) nach Auslösen bei Zurückziehen der Kolbenstange (18) sich von der Kolbenstange (18) löst, dass vorzugsweise nach Betätigen des Auslöseelementes (10) dieses in der Injektorhülse (17) verbleibt und dass vorzugsweise die Kolbenstange (18) mindestens einmal dichtend abgequetscht ist.

## Claims

1. An injection device for the needle-free injection of a medium, comprising an injector and an ampoule unit, wherein the ampoule unit (2) has a basic body (4) for storing the medium (3) to be injected and which has a casing (5) exerting compressive forces on the basic body (4), wherein a nozzle (13), which forms an extension of the basic body, is arranged at the longitudinal axial end of the ampoule unit that forms an outlet for the medium (3) to be injected, **characterised in that** the casing (5) completely surrounds the basic body (4) in the longitudinal axial direction and partially surrounds the nozzle (13) so that the nozzle (13) abuts against the inner walls of the casing (5) in a form-fitting and non-positive manner and forms a press fit, **in that** the compressive forces of the casing (5) at least counterbalance the internal stresses arising during the manufacture of the basic body, **in that** the basic body is made of glass, preferably borosilicate glass, **in that** the piston rod (18) is hollow and, after the ampoule has been filled with the medium, the piston rod is squeezed at at least one point.

2. An injection device according to claim 1, **characterised in that** the casing (5) exerts on the basic body at least up to 100% of the compressive forces arising during injection.

3. An injection device according to either one of claims 1 and 2, **characterised in that** the casing (5) is shrunk onto the basic body (4) or is applied by a casting process, by polymerisation or by means of a press fit.

4. An injection device according to any one of claims 1 to 3, **characterised in that** the casing (5) is made of plastic or metal, the basic body (4) is preferably made of glass and the casing (5) is preferably made of polyamide, a fibre composite or an MCP alloy.

5. An injection device according to any one of claims 1 to 4, **characterised by** an injector sleeve (17) for the needle-free injection of a medium (3), comprising the injector with a spring drive element (6) for driving a propulsion element (7) for expelling the medium (3) for injection of the same, wherein in the spring-loaded state the injector is formed so that, with respect to the tension of the spring drive element, exclusively the spring drive element (6), the propulsion element (7), a locking element (8) and a thrust bearing means (9) are acted upon by the spring tension force, wherein the injector sleeve (17) is free of spring tension forces on account of the thrust bearing means (9) being independent of the injector sleeve, the propulsion element (7) that is preferably tensioned by the spring drive element (6) is releasably secured/securable by a locking element (8), and the forces generated by the spring drive element (6) are absorbed by a thrust bearing means (9), and further preferably the locking element (8) is a pin-type element which engages the propulsion element (7) so as to secure the latter, and the locking element (8) is preferably formed as a lever, and/or the propulsion element (7) is secured against an undercut, and a release element (10) for releasing the propulsion element (7) that is tensioned by the spring drive element (6) is preferably actuatable in the injection direction of the medium (3), wherein the propulsion element (7) is preferably releasable by pivoting or rotating the locking element (8), the release element (10) is preferably wedge-shaped and the release element (10) runs onto the locking element (8) upon actuation of the release element (10) in the injection direction.

6. An injection device according to any one of claims 1 to 5, **characterised in that** the ampoule unit is attachable to and/or detachable from the injector, **in that** the ampoule unit is preferably attachable to the injector in a non-positive manner so as to bring the injection device into a pre-injection state, **in that** the attachability and detachability is preferably effected by means of a thread or an annular snap-locking connection.

7. An injection device according to any one of claims 1 to 6, **characterised in that** the ampoule unit is prefilled with the medium to be injected, **in that** the ampoule unit is preferably prefilled in a gas-free or virtually gas-free manner.

8. An injection device according to any one of claims 1 to 7, **characterised in that** the injector has a securing element (11) for securing the release element (10) during transportation and/or against unintentional release, **in that** the securing element (11) preferably is of a ring-type formation, **in that** the injector and/or the release element (10) preferably has a groove (12) for receiving the securing element (11), **in that** the injection device preferably has a longitudinally movable stopper element (16) closing one longitudinal axial end, **in that** the stopper element (16) preferably expels the medium from the ampoule unit during injection, **in that** the attachability and detachability is preferably effected by means of a thread or an annular snap-locking connection, **in that** a nozzle (13) is preferably arranged at the longitudinal axial end of the ampoule unit that forms an outlet for the medium (3) to be injected, **in that** the longitudinal axial end of the ampoule unit that forms an outlet for the medium (3) to be injected is preferably covered by a cap (14), **in that** a resilient sealing member (15) is preferably arranged between the basic body (4) and the nozzle (13), **in that** the resilient sealing member (15) is preferably made of silicone, **in that** the nozzle (13) is preferably made of steel, **in that** the nozzle (13) preferably has one or at least two outlet openings, **in that** the nozzle (13) preferably forms a sealing press fit with the nozzle screw connection (19), **in that** a piston rod (18) driving the stopper element (16) is preferably hollow, **in that** the stopper element (16) preferably has a material recess corresponding to the hollowness of the piston rod (18), **in that** the stopper element (16) preferably detaches itself from the piston rod (18) after release when the piston rod (18) is retracted, **in that** the release element (10) preferably remains in the injector sleeve (17) after actuation of the release element (10) and **in that** the piston rod (18) is preferably squeezed at least once so as to form a seal.

## Revendications

1. Dispositif d'injection servant à l'injection sans aiguille d'un milieu, avec un dispositif injecteur et une unité ampoule, l'unité ampoule (2) présentant un corps de base (4) conservant le milieu à injecter (3), qui présente une enveloppe (5) exerçant des efforts de pression sur le corps de base (4), une buse (13) étant agencée comme sortie du milieu à injecter (3) au niveau de l'extrémité axiale longitudinale de l'unité ampoule, laquelle buse se trouve dans le prolongement du corps de base,
**caractérisé en ce que** l'enveloppe (5) entoure entièrement le corps de base (4) dans le sens axial longitudinal et entoure partiellement la buse (13) de sorte que la buse (13) s'appuie par correspondance de forme et par force contre les parois intérieures de l'enveloppe (5) et forme un ajustement de précision, **en ce que** les efforts de pression de l'enveloppe (5) compensent au moins les contraintes internes générées lors de la fabrication du corps de base, le corps de base est en verre, de préférence en verre borosilicaté, **en ce que** la tige de piston (18) est creuse et après le remplissage de l'ampoule de milieu, la tige piston est écrasée à au moins un endroit.

2. Dispositif d'injection selon la revendication 1,
**caractérisé en ce que** l'enveloppe (5) exerce au moins jusqu'à 100 % des efforts de pression survenant lors de l'injection sur le corps de base.

3. Dispositif d'injection selon l'une quelconque des revendications 1 à 2,
**caractérisé en ce que** l'enveloppe (5) est frettée sur le corps de base (4) ou appliquée selon le procédé de moulage, par polymérisation ou à l'aide d'un ajustement serré.

4. Procédé d'injection selon l'une quelconque des revendications 1 à 3,
**caractérisé en ce que** l'enveloppe (5) est en plastique ou en métal, le corps de base (4) est de préférence en verre et l'enveloppe (5) est de préférence en polyamide, en matériau renforcé par des fibres ou en alliage MCP.

5. Dispositif d'injection selon l'une quelconque des revendications 1 à 4,
**caractérisé par** une douille d'injecteur (17) servant à l'injection sans aiguille d'un milieu (3), le dispositif injecteur comprenant un élément d'entraînement par ressort (6) servant à entraîner un élément de commande (7) pour faire sortir le milieu (3) afin de l'injecter, le dispositif injecteur étant réalisé à l'état de tension du ressort de telle sorte que lorsque l'élément d'entraînement par ressort est tendu, seuls l'élément d'entraînement par ressort (6), l'élément de commande (7), l'élément de blocage (8) et le dispositif de palier de butée (9) sont soumis à la contrainte du ressort, la douille d'injecteur (17) étant exempte de contraintes du ressort grâce au dispositif de palier de butée (9) indépendant de la douille d'injecteur, l'élément de commande (7) précontraint de préférence par le biais de l'élément d'entraînement par ressort (6) est arrêté / peut être arrêté de manière à pouvoir être libéré par le biais d'un élément de blocage (8) et les efforts générés par l'élément d'entraînement par ressort (6) sont absorbés par un dispositif de palier de butée (9) et de préférence en outre l'élément de blocage (8) est un élément de type tige, qui s'applique au niveau de l'élément de commande (7) en l'arrêtant et de préférence l'élément de blocage (8) est réalisé sous forme de levier et/ou arrête l'élément de commande (7) au niveau d'une contre-dépouille et de préférence un élément de déclenchement (10) servant à libérer l'élément de commande (7) précontraint par le biais de l'élément d'entraînement par ressort (6) peut être actionné dans le sens d'injection du milieu (3), en ce que de préférence la libération de l'élément de commande (7) peut être effectuée par pivotement ou rotation de l'élément de blocage (8), en ce que de préférence l'élément de déclenchement (10) est réalisé en forme de coin, en ce que lors de l'actionnement de l'élément de déclenchement (10) dans le sens d'injection, celui-ci vient se placer sur l'élément de blocage (8).

6. Dispositif d'injection selon l'une quelconque des revendications 1 à 5,
**caractérisé en ce que** l'unité ampoule peut être montée et/ou démontée au niveau du dispositif injecteur, **en ce que** de préférence l'unité ampoule peut être montée par force sur le dispositif injecteur, amenant le dispositif d'injection dans un état de pré-injection, **en ce que** de préférence la capacité de montage et de démontage est réalisée à l'aide d'un filetage ou d'un assemblage par encliquetage annulaire.

7. Dispositif d'injection selon l'une quelconque des revendications 1 à 6,
**caractérisé en ce que** l'unité ampoule est pré-remplie de milieu à injecter, **en ce que** de préférence l'unité ampoule est pré-remplie sans gaz ou pratiquement sans gaz.

8. Dispositif d'injection selon l'une quelconque des revendications 1 à 7,
**caractérisé en ce que** le dispositif injecteur présente un élément de sécurité (11) servant à bloquer l'élément de déclenchement (10) lors du transport et/ou contre un déclenchement non intentionnel, **en ce que** de préférence l'élément de sécurité (11) est réalisé de manière annulaire, **en ce que** de préférence le dispositif injecteur et/ou l'élément de déclenchement (10) présente une rainure (12) servant à recevoir l'élément de sécurité (11), **en ce que** de préférence le dispositif d'injection présente un bouchon (16) déplaçable dans le sens longitudinal, fermant un côté axial longitudinal, **en ce que** de préférence le bouchon (16) fait sortir le milieu de l'unité ampoule lors de l'injection, **en ce que** de préférence la capacité de montage et de démontage est réalisée à l'aide d'un filetage ou d'un assemblage par encliquetage annulaire, **en ce que** de préférence une buse (13) est agencée comme sortie du milieu à injecter (3) au niveau de l'extrémité axiale longitudinale de l'unité ampoule, **en ce que** de préférence l'extrémité axiale longitudinale de l'unité ampoule comme sortie du milieu à injecter (3) est recouverte par un capuchon (14), **en ce que** de préférence un élément d'étanchéité élastique (15) est agencé entre le corps de base (14) et la buse (13), **en ce que** de préférence l'élément d'étanchéité élastique (15) est en silicone, **en ce que** de préférence la buse (13) est en acier, **en ce que** de préférence la buse (13) présente une ou au moins deux ouvertures de sortie, **en ce que** de préférence la buse (13) forme un ajustement de précision étanche avec le vissage de buse (19), **en ce que** de préférence une tige de piston (18) entraînant le bouchon (16) est creuse, **en ce que** de préférence le bouchon (16) présente un évidement de matière correspondant à la cavité de la tige de piston (18), **en ce que** de préférence le bouchon (16) se détache de la tige de piston (18) après déclenchement lors du retrait de la tige de piston (18), **en ce que** de préférence après actionnement de l'élément de déclenchement (10) celui-ci reste dans la douille d'injecteur (17) et **en ce que** de préférence la tige de piston (18) est écrasée au moins une fois de manière étanche.
